(19) Europäisches Patentamt
European Patent Office
Office européen des brevets

(11) Veröffentlichungsnummer: **0 160 208**
**A1**

# (12) EUROPÄISCHE PATENTANMELDUNG

(21) Anmeldenummer: **85103499.1**

(22) Anmeldetag: **25.03.85**

(51) Int. Cl.⁴: **C 07 D 251/38**
**C 07 D 251/46, A 01 N 43/64**

(30) Priorität: **06.04.84 DE 3412946**

(43) Veröffentlichungstag der Anmeldung:
**06.11.85 Patentblatt 85/45**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Dickoré, Karlfried, Dr.**
**Nicolai-Hartmann-Strasse 19**
**D-5090 Leverkusen 1(DE)**

(72) Erfinder: **Eue, Ludwig, Dr.**
**Paul-Klee-Strasse 36**
**D-5090 Leverkusen 1(DE)**

(72) Erfinder: **Schmidt, Robert R. Dr.**
**Im Waldwinkel 110**
**D-5060 Bergisch-Gladbach 2(DE)**

(72) Erfinder: **Santel, Hans-Joachim, Dr.**
**Gerstenkamp 19**
**D-5000 Koeln 80(DE)**

## (54) 3-(2,2-Tetramethylen-propyl)-1,3,5-triazin-2,4-dione.

(57) Die Erfindung betrifft neue 3-(2,2-Tetramethylenpropyl)-1,3,5-triazin-2,4-dione der allgemeinen Formel

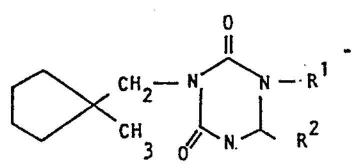

(I)

in welcher

$R^1$ für Alkyl, für Amino oder für einen Rest $-N=C\begin{smallmatrix}R^3\\R^4\end{smallmatrix}$

steht, wobei $R^3$ und $R^4$ unabhängig voneinander für Wasserstoff, für Alkyl oder für gegebenenfalls substituiertes Aryl stehen, oder gemeinsam für einen zweifach verknüpften Alkandiylrest stehen, und

$R^2$ für Alkylthio, Alkylamino, Alkenylamino, Cycloalkylamino oder Dialkylamino steht, verschiedene Verfahren zu ihrer Herstellung und ihre Verwendung als Herbizide.

EP 0 160 208 A1

Croydon Printing Company Ltd

BAYER AKTIENGESELLSCHAFT 5090 Leverkusen, Bayerwerk
Konzernverwaltung RP
Patentabteilung Bi/ABc
Ib

# 3-(2,2-Tetramethylen-propyl)-1,3,5-triazin-2,4-dione

Die Erfindung betrifft neue 3-(2,2-Tetramethylen-propyl)-1,3,5-triazin-2,4-dione, mehrere Verfahren zu ihrer Herstellung und ihre Verwendung als Herbizide.

Es ist bereits bekannt, daß bestimmte 1,3,5-Triazin-2,4-dione, wie beispielsweise das 1-Amino-6-ethylthio-3-neopentyl-1,2,3,4-tetrahydro-1,3,5-triazin-2,4-dion, herbizide Eigenschaften besitzen (vgl. z.B. DK-PS 136.067 /Le A 14 692/).

Die herbizide Wirkung dieser Verbindungen gegenüber Schadpflanzen, ebenso wie deren Verträglichkeit gegenüber wichtigen Kulturpflanzen, ist jedoch nicht immer in allen Anwendungsbereichen völlig zufriedenstellend.

Es wurden neue 3-(2,2-Tetramethylen-propyl)-1,3,5-triazin-2,4-dione der allgemeinen Formel (I),

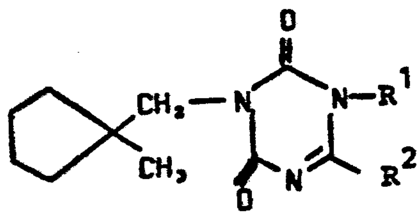

(I)

in welcher

$R^1$ für Alkyl, für Amino oder für einen Rest

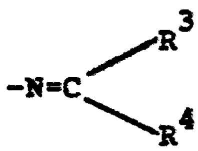

steht, wobei $R^3$ und $R^4$ unabhängig voneinander für Wasserstoff, für Alkyl oder für gegebenenfalls substituiertes Aryl stehen, oder gemeinsam für einen zweifach verknüpften Alkandiylrest stehen, und

$R^2$ für Alkylthio, Alkylamino, Alkenylamino, Cycloalkylamino oder Dialkylamino steht,

gefunden.

Weiterhin wurde gefunden, daß man die neuen 3-(2,2-Tetramethylen-propyl)-1,3,5-triazin-2,4-dione der allgemeinen Formel (I) erhält, wenn man

(a) Isothioharnstoff-Derivate der Formel (II),

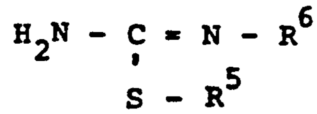

(II)

in welcher

$R^5$ für Alkyl steht und

$R^6$ für Alkyl oder für gegebenenfalls substituiertes Alkylidenimino bzw. Cycloalkylidenimino steht,

oder deren Hydrosalze mit (2,2-Tetramethylen-propyl)-amin-Derivaten der Formel (III),

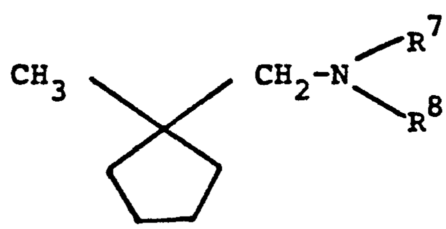

(III)

in welcher

$R^7$ und $R^8$ unabhängig voneinander jeweils (a) für gegebenenfalls substituiertes Aryloxycarbonyl oder (b) für Chlorcarbonyl stehen,

gegebenenfalls in Gegenwart eines Verdünnungsmittels und gegebenenfalls in Gegenwart eines Säurebindemittels umsetzt oder wenn man

(b) die nach Verfahren (a) erhältlichen 1-Alkylidenimino-3-(2,2-tetramethylen-propyl)-1,3,5-triazin-2,4-dione der Formel (Ia),

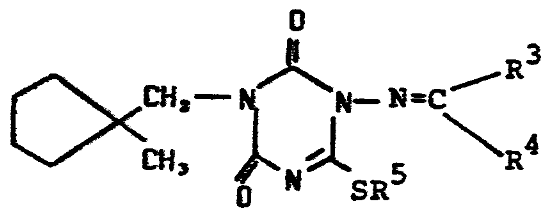

(Ia)

in welcher

$R^3$, $R^4$ und $R^5$ die oben angegebene Bedeutung haben,

in prinzipiell bekannter Weise mit wäßrigen Säuren, gegebenenfalls in Gegenwart eines Verdünnungsmittels und gegebenenfalls in Gegenwart eines Katalysators, umsetzt zu den 1-Amino-3-(2,2-tetramethylen-propyl)-1,3,5-triazin-2,4-dionen der Formel (Ib),

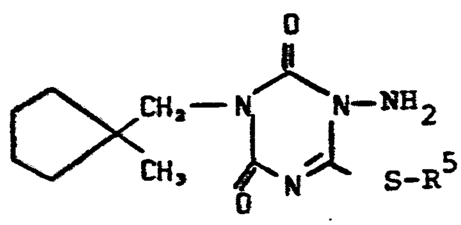

(Ib)

in welcher

$R^5$ die oben angegebene Bedeutung hat,

oder wenn man

(c) die nach Verfahren (a) oder (b) erhältlichen 6-Alkylthio-3-(2,2-tetramethylen-propyl)-1,3,5-triazin-2,4-dione der Formel (Ic),

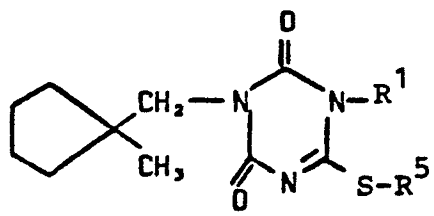

(Ic)

in welcher

$R^1$ und $R^5$ die oben angegebene Bedeutung haben,

mit Aminen der Formel (IV),

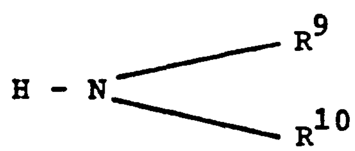

(IV)

in welcher

$R^9$ für Wasserstoff, Alkyl, Alkenyl oder Cycloalkyl steht und

$R^{10}$ für Alkyl steht,

gegebenenfalls in Gegenwart eines Verdünnungsmittels umsetzt zu den 6-Amino-3-(2,2-tetramethylen-propyl)-1,3,5-triazin-2,4-dionen der Formel (Id),

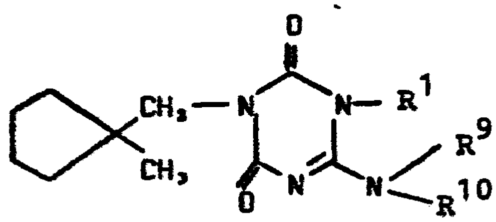

(Id)

in welcher

$R^1$, $R^9$ und $R^{10}$ die oben angegebene Bedeutung haben.

Schließlich wurde gefunden, daß die neuen 3-(2,2-Tetramethylen-propyl)-1,3,5-triazin-2,4-dione der allgemeinen Formel (I) herbizide, insbesondere selektiv-herbizide Eigenschaften besitzen.

Überraschenderweise zeigen die erfindungsgemäßen 3-(2,2-Tetramethylen-propyl)-1,3,5-triazin-2,4-dione der Formel (I) eine bessere herbizide Wirkung gegen Schadpflanzen und gleichzeitig eine verbesserte Verträglichkeit gegenüber wichtigen Kulturpflanzen im Vergleich zu den aus dem Stand der Technik bekannten 1,3,5-Triazin-2,4-dionen (wie beispielsweise 1-Amino-6-ethylthio-3-(2,2-dimethyl-propyl)-1,3,5-triazin-2,4-dion), welche chemisch und wirkungsmäßig naheliegende Verbindungen sind.

Die neuen 3-(2,2-Tetramethylen-propyl)-1,3,5-triazin-2,4-dione sind durch die Formel (I) allgemein definiert. Bevorzugt sind Verbindungen der Formel (I), bei welchen

$R^1$ für Amino, für geradkettiges oder verzweigtes Alkyl mit bis zu 4 Kohlenstoffatomen oder für einen Rest

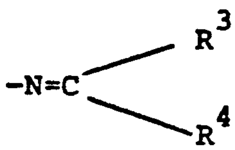 steht, wobei $R^3$ und $R^4$ jeweils

unabhängig voneinander für Wasserstoff, für geradkettiges oder verzweigtes Alkyl mit bis zu 6 Kohlenstoffatomen oder für einfach oder mehrfach, gleich oder verschieden durch Halogen, Cyano, Nitro, sowie jeweils geradkettiges oder verzweigtes Alkyl, Alkoxy oder Alkylthio mit jeweils bis zu 4 Kohlenstoffatomen substituiertes Phenyl stehen oder gemeinsam für einen zweifach gebundenen Alkandiylrest mit 4 bis 6 Kohlenstoffatomen stehen und

$R^2$ für jeweils geradkettiges oder verzweigtes Alkylthio, Alkylamino oder Dialkylamino mit jeweils bis zu 6 Kohlenstoffatomen in den einzelnen Alkylteilen steht.

Besonders bevorzugt sind Verbindungen der Formel (I), bei welchen

$R^1$ für Methyl, Ethyl, Amino oder einen der Reste

$-N{=}C(CH_3)_2$, $-N{=}CH{-}C(CH_3)_3$, $-N{=}CH{-}C_6H_5$, 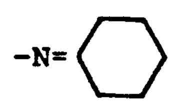 ,

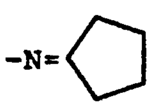 oder 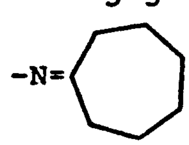 steht und

$R^2$ für Methylthio, Ethylthio, n- und i-Propylthio, n-, i-, s- und t-Butylthio, Methylamino, Ethylamino, Dimethylamino oder Diethylamino steht.

Im einzelnen seien die folgenden 3-(2,2-Tetramethylen-propyl)-1,3,5-triazin-2,4-dione der allgemeinen Formel (I) genannt:

<u>Tabelle 1</u>

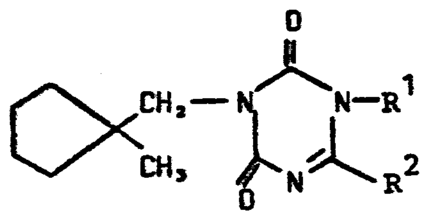

| $R^1$ | $R^2$ |
|---|---|
| $CH_3$ | $-SCH_3$ |
| $CH_3$ | $-SC_2H_5$ |
| $CH_3$ | $-NH-CH_3$ |
| $CH_3$ | $-N(CH_3)_2$ |
| $CH_3$ | 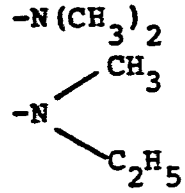 |
| $CH_3$ | $-NH-C_2H_5$ |
| $CH_3$ | $-N(C_2H_5)_2$ |
| $CH_3$ | -NH-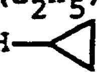 |
| $CH_3$ | $-NH-CH_2-CH=CH_2$ |
| $NH_2$ | $-SCH_3$ |
| $NH_2$ | $-SC_2H_5$ |
| $NH_2$ | $-NH-CH_3$ |
| $NH_2$ | $-N(CH_3)_2$ |

| $R^1$ | $R^2$ |
|---|---|
| $-NH_2$ | 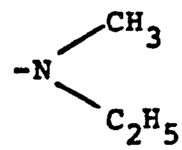 |
| $-NH_2$ | $-NH-C_2H_5$ |
| $-NH_2$ | $-N(C_2H_5)_2$ |
| $-NH_2$ | -NH-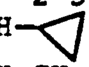 |
| $-NH_2$ | $-NH-CH_2-CH = CH_2$ |
| $-N = C(CH_3)_2$ | $-SCH_3$ |
| $-N = C(CH_3)_2$ | $-SC_2H_5$ |
| $-N = C(CH_3)_2$ | $-NH-CH_3$ |
| $-N = C(CH_3)_2$ | $-N(CH_3)_2$ |
| $-N = CH-CH(CH_3)_2$ | $-SCH_3$ |
| $-N = CH-CH(CH_3)_2$ | $-SC_2H_5$ |
| $-N = CH-CH(CH_3)_2$ | $-NH-CH_3$ |
| $-N = CH-CH(CH_3)_2$ | $-N(CH_3)_2$ |
| -N 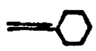 | $-SCH_3$ |
| -N 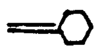 | $- SC_2H_5$ |
| -N 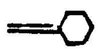 | $-NH-CH_3$ |
| -N 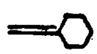 | $-N(CH_3)_2$ |
| $-N = CH-C_6H_5$ | $-SCH_3$ |
| $-N = CH-C_6H_5$ | $-SC_2H_5$ |
| $-N = CH-C_6H_5$ | $-NH-CH_3$ |
| $-N = CH-C_6H_5$ | $-N(CH_3)_2$ |

Verwendet man als Ausgangsstoffe beispielsweise Aceton-(S-methyl-isothiosemicarbazon) und N,N-Bisphenoxycarbonyl-(2,2-tetramethylen-propyl)-amin, so läßt sich der Reaktionsablauf des erfindungsgemäßen Verfahrens (a) durch das folgende Formelschema darstellen:

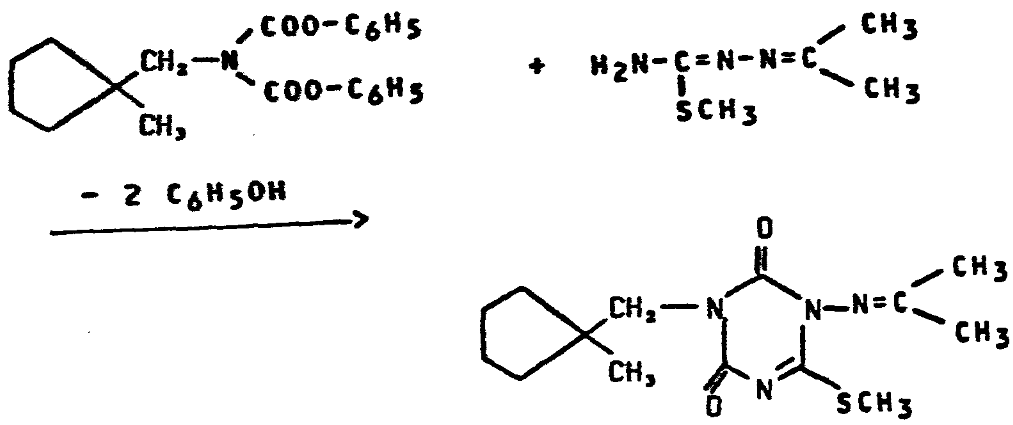


Verwendet man beispielsweise Aceton-(S-ethyl-isothiosemicarbazon)-hydrobromid, N,N-Bischlorcarbonyl-(2,2-tetramethylen-propyl)-amin und Triethylamin als Ausgangsstoffe, so läßt sich der Reaktionsablauf des erfindungsgemäßen Verfahrens (a) durch das folgende Formelschema darstellen:

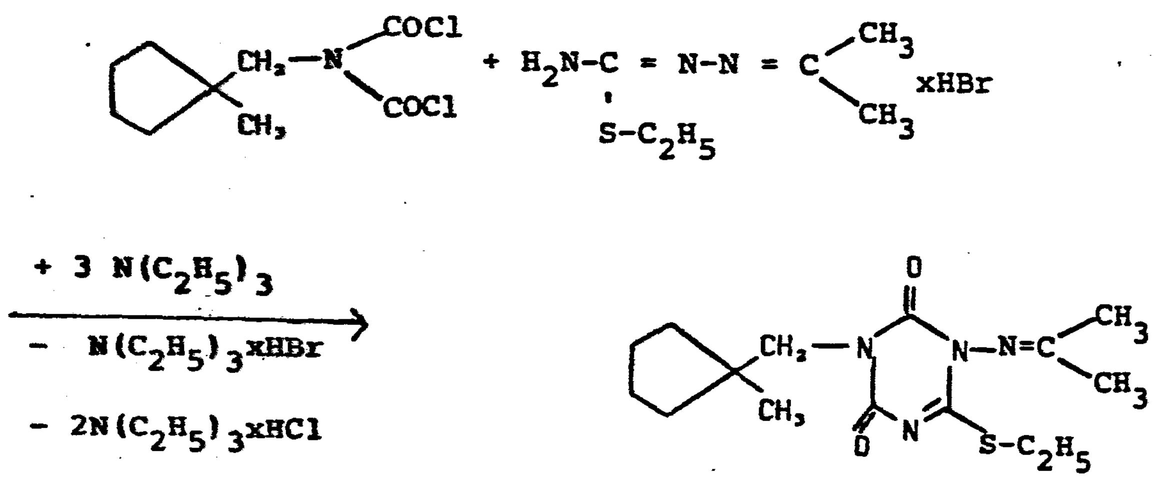

Verwendet man als Ausgangsstoffe beispielsweise 3-(2,2-Tetramethylen-propyl)-6-methylthio-1-isopropylidenimino-1,2,3,4-tetrahydro-1,3,5-triazin-2,4-dion und eine wäßrige Säure, so läßt sich der Reaktionsablauf des erfindungsgemäßen Verfahrens (b) durch das folgende Formelschema darstellen:

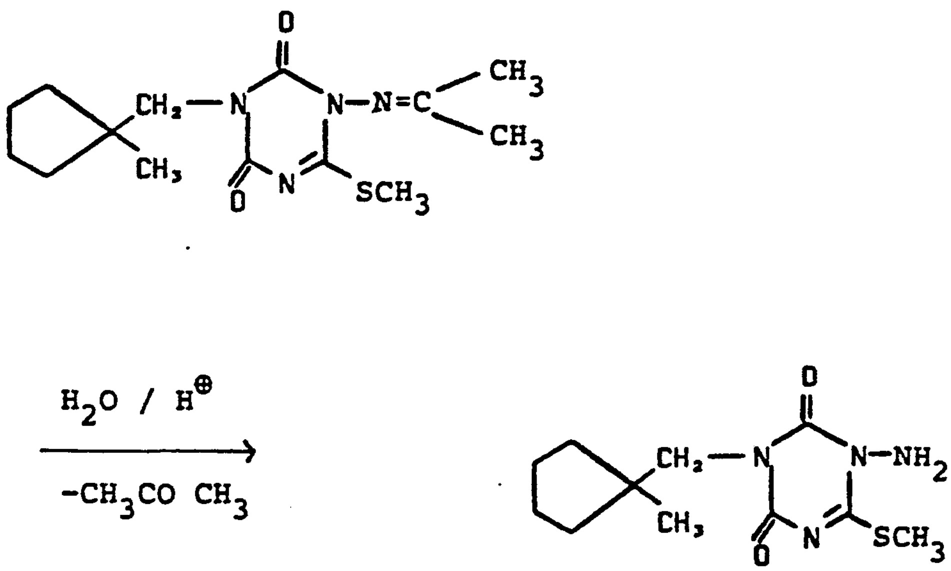


Verwendet man als Ausgangsstoffe beispielsweise 3-(2,2-Tetramethylen-propyl)-3-ethylthio-1-methyl-1,2,3,4-tetrahydro-1,3,5-triazin-2,4-dion und Dimethylamin, so läßt sich der Reaktionsablauf des erfindungsgemäßen Verfahrens (c) durch das folgende Formelschema darstellen:

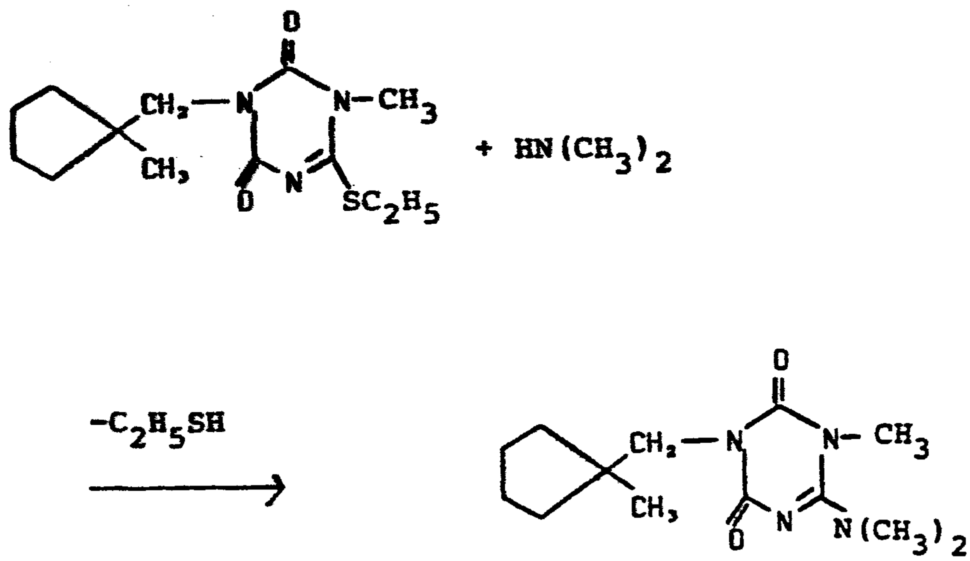


Die zur Durchführung des erfindungsgemäßen Verfahrens (a) als Ausgangsstoffe benötigten Isothioharnstoff-Derivate sind durch die Formel (II) allgemein definiert. In dieser Formel (II) steht $R^5$ vorzugsweise für Alkyl mit 1-6 C-Atomen; $R^6$ steht vorzugsweise für Alkyl mit 1-4 C-Atomen, Alkylidenimino mit 2-7 C-Atomen, Cycloalkylidenimino mit 5-7 C-Atomen oder Benzylidenimino, insbesondere für Methyl, Ethyl oder einen der Reste $-N=C(CH_3)_2$, $-N=CH-C_6H_5$, $-N=CH-C(CH_3)_3$ oder -N=⬡.

Die Isothioharnstoff-Derivate der Formel (II) sind bekannt (vgl. z. B. DE-OS 31 47 735).

Die weiterhin zur Durchführung des erfindungsgemäßen Verfahrens (a) als Ausgangsstoffe benötigten (2,2-Tetramethylen-propyl)-amin-Derivate sind durch die Formel (III) allgemein definiert. In dieser Formel (III) stehen

$R^7$ und $R^8$ vorzugsweise gleichzeitig für Phenoxycarbonyl oder Chlorcarbonyl.

Die (2,2-Tetramethylen-propyl)-amin-Derivate der Formel (IIIa)

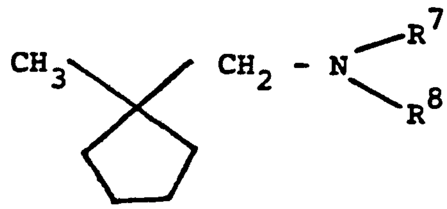

(IIIa)

in welcher $R^7$ und $R^8$ für gegebenenfalls substituiertes Aryloxycarbonyl stehen, sind noch nicht bekannt. Man erhält die Verbindungen der Formel (IIIa), wenn man (2,2-Tetramethylen-propyl)-amin der Formel (V)

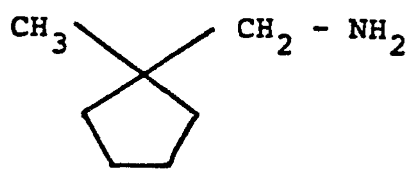

(V)

mit allgemein bekannten Chlorameisensäurearylestern der Formel (VIa)

$$R^7 - Cl \qquad (VIa)$$

in welcher

$R^7$ für gegebenenfalls substituiertes Aryloxycarbonyl steht,

zunächst in einer ersten Stufe, gegebenenfalls in Gegenwart eines Verdünnungsmittels, wie beispielsweise Methylenchlorid, und gegebenenfalls in Gegenwart eines Säurebindemittels, wie beispielsweise Natriumhydroxid, bei Temperaturen zwischen -20°C und +30°C umsetzt und die so erhaltenen monoacylierten (2,2-Tetramethylen-propyl)-amine der Formel (VII)

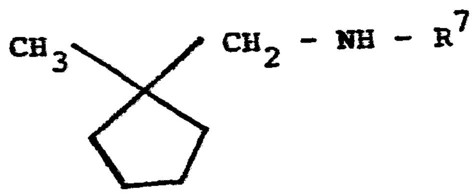

(VII)

in welcher

$R^7$ die oben angegebene Bedeutung hat,

in einer zweiten Stufe mit allgemein bekannten Chlorameisensäureestern der Formel (VIb)

$$R^8 - Cl \qquad (VIb)$$

in welcher

$R^8$ für gegebenenfalls substituiertes Aryloxycarbonyl steht,

gegebenenfalls in Gegenwart eines Verdünnungsmittels wie beispielsweise Methylenchlorid bei Temperaturen zwischen +80°C und +200°C umsetzt.

Das (2,2-Tetramethylen-propyl)-amin-Derivat der Formel (IIIb),

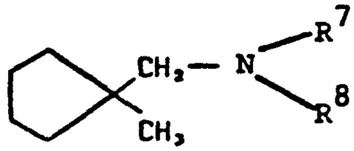

(IIIb)

in welcher $R^7$ und $R^8$ für Chlorcarbonyl stehen, ist ebenfalls noch nicht bekannt.

Man kann diese Verbindung (IIIb) herstellen, indem man das oben genannte (2,2-Tetramethylen-propyl)-amin der Formel (V) über das Formamid (VIII) durch Umsetzung mit Chlorcarbonylsulfenchlorid (IX) in das Dithiazolidindion (X) überführt, welches chlorolytisch zu (IIIb) gespalten wird (vgl. BE-PS 682 820; Angew. Chem. 82 (1970), S. 63-67; Synthesis 1970, S. 542-543; DE-OS 2 254 200):

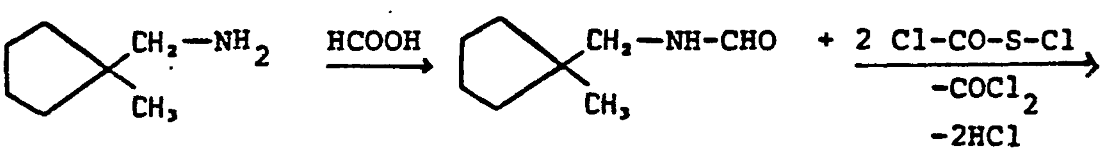


(V) (VIII) (IX)

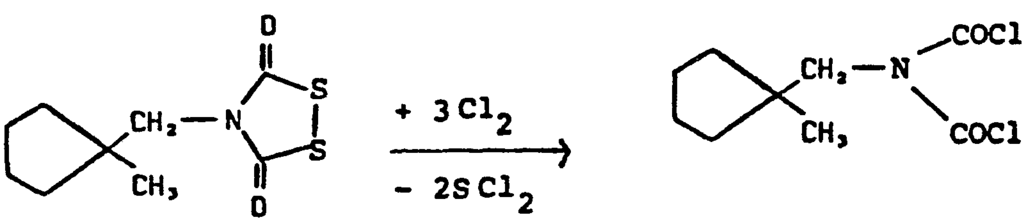


(IIIb)

Die zur Durchführung des erfindungsgemäßen Verfahrens (b) als Ausgangsstoffe benötigten 1-Alkylidenimino-3-(2,2-tetramethylen-propyl)-1,3,5-triazin-2,4-dione sind durch die Formel (Ia) allgemein definiert. In dieser Formel (Ia) stehen $R^3$, $R^4$ und $R^5$ vorzugsweise für diejenigen Reste, die schon bei der Beschreibung der erfindungsgemäßen Stoffe der Formel (I) und der Vorprodukte der Formel (II) als bevorzugt für diese Substituenten genannt wurden.

Die 1-Alkylidenimino-3-(2,2-tetramethylen-propyl)-1,3,5-triazin-2,4-dione der Formel (Ia) sind erfindungsgemäße Verbindungen und erhältlich mit Hilfe des erfindungsgemäßen Verfahrens (a).

Die zur Durchführung des erfindungsgemäßen Verfahrens (c) als Ausgangsstoffe benötigten 6-Alkylthio-3-(2,2-tetramethylen-propyl)-1,3,5-triazin-2,4-dione sind durch die Formel (Ic) allgemein definiert. In dieser Formel (Ic) stehen $R^1$ und $R^5$ vorzugsweise für diejenigen Reste, die schon bei der Beschreibung der erfindungsgemäßen Stoffe der Formel (I) und der Vorprodukte der Formel (II) vorzugsweise für diese Substituenten genannt wurden.

Die 6-Alkylthio-3-(2,2-tetramethylen-propyl)-1,3,5-triazin-2,4-dione sind erfindungsgemäße Verbindungen und erhältlich mit Hilfe der erfindungsgemäßen Verfahren (a) oder (b).

Die weiterhin zur Durchführung des erfindungsgemäßen Verfahrens (c) als Ausgangsstoffe benötigten Amine sind

durch die Formel (IV) allgemein definiert. In dieser Formel (IV) stehen $R^9$ und $R^{10}$ unabhängig voneinander vorzugsweise für Wasserstoff oder für geradkettiges oder verzweigtes Alkyl mit bis zu 6 Kohlenstoffatomen, insbesondere für Methyl oder Ethyl.

Die Amine der Formel (IV) sind allgemein bekannte Verbindungen der organischen Chemie.

Das zur Herstellung der Vorprodukte der Formel (III) als Ausgangsstoff benötigte (2,2-Tetramethylen-propyl)-amin der Formel (V) ist noch nicht bekannt. Es läßt sich nach prinzipiell bekannten Verfahren durch katalytische Hydrierung des bekannten (vgl. J. Org. Chem. 43, S. 1044 (1978); DE-OS 3115 976) 1-Methyl-cyclopentyl-carbonitrils herstellen.

Die weiterhin zur Herstellung der Vorprodukte der Formel (III) als Ausgangsstoffe benötigten Chlorameisensäurearylester der Formeln (VIa) und (VIb) sind allgemein bekannte Verbindungen der organischen Chemie.

Als Verdünnungsmittel zur Durchführung des erfindungsgemäßen Verfahrens (a) kommen inerte organische Lösungsmittel in Frage. Vorzugsweise verwendet man aliphatische oder aromatische, gegebenenfalls halogenierte Kohlenwasserstoffe, wie beispielsweise Benzin, Benzol, Toluol, Xylol oder Chlorbenzol, Methylenchlorid, Chloroform oder Tetrachlorkohlenstoff oder Alkohole, wie Methanol, Ethanol, n- und i-Propanol oder n- und i-Butanol. Man kann jedoch auch ohne Verwendung von Lösungsmitteln in der Schmelze der Ausgangsprodukte arbeiten.

Als Säurebindemittel kommen bei der Durchführung des erfindungsgemäßen Verfahrens (a) alle üblicherweise verwendbaren organischen oder anorganischen Basen in Frage. Vorzugsweise verwendet man Alkalimetallhydroxide, -carbonate oder -hydrogencarbonate wie beispielsweise Natriumhydroxid oder Kaliumhydroxid, Natriumcarbonat, Kaliumcarbonat oder Natriumhydrogencarbonat oder tertiäre Amine wie Triethylamin, N,N-Dimethylanilin, 4-Dimethylaminopyridin, Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man zwischen +20°C und +200°C, vorzugsweise zwischen +50°C und +150°C.

Zur Durchführung des erfindungsgemäßen Verfahrens (a) setzt man pro Mol Isothioharnstoff-Derivat der Formel (II) im allgemeinen 0,8 bis 1,5 Mol, vorzugsweise äquimolare Mengen an (2,2-Tetramethylen-propyl)-amin-Derivaten der Formel (IIIa) ein und bei Verwendung der Bischlorcarbonyl-amin-Derivate (IIIb) bzw. von Isothioharnstoff-Hydrosalzen als Ausgangsstoffe im allgemeinen 0,8 bis 1,5 Mol, vorzugsweise äquimolare Mengen an Säurebindemittel ein. Das bei dieser Cyclisierungsreaktion, falls (IIIa) eingesetzt wird, freiwerdende gegebenenfalls substituierte Phenol wird entweder im Vakuum abdestilliert oder extraktiv durch Behandlung mit wäßrigen Basen,

wie beispielsweise Natronlauge entfernt. Die Aufarbeitung und Isolierung der Reaktionsprodukte der Formel (Ia) erfolgt nach üblichen Methoden.

Als Verdünnungsmittel zur Durchführung des erfindungsgemäßen Verfahrens (b) kommen ebenfalls inerte organische Lösungsmittel in Frage; vorzugsweise verwendet man Kohlenwasserstoffe wie Benzin, Benzol, Toluol oder Xylol oder Alkohole wie Methanol, Ethanol, Propanol oder Butanol.

Als Spaltungsreagenz ebenso wie als Katalysator kommen anorganische oder organische Säuren in Frage. Vorzugsweise verwendet man Mineralsäuren in wäßriger Lösung wie beispielsweise Salzsäure oder Schwefelsäure oder organische Sulfonsäuren wie beispielsweise p-Toluolsulfonsäure.

Die Reaktionstemperaturen können bei der Durchführung des erfindungsgemäßen Verfahrens (b) ebenfalls in einem größeren Bereich variiert werden. Im allgemeinen arbeitet man zwischen +20°C und +150°C, vorzugsweise zwischen +40°C und +120°C.

Zur Durchführung des erfindungsgemäßen Verfahrens (b) setzt man pro Mol 1-Alkylidenimino-3-(2,2-tetramethylenpropyl)-1,3,5-triazin-2,4-dion der Formel (Ia) 0,01 bis 0,5 Mol an anorganischer oder organischer Säure ein. Die Aufarbeitung und Isolierung der Endprodukte der Formel (Ib) erfolgt nach bekannten und üblichen Verfahren

(vgl. z.B. DE-OS 22 54 200, US-PS 4 056 527, DE-OS 31 47 735, DE-OS 32 41 114).

Als Verdünnungsmittel zur Durchführung des erfindungsgemäßen Verfahrens (c) kommen wäßrige oder organische Lösungsmittel in Frage. Vorzugsweise verwendet man Alkohole, Wasser oder Wasser-Alkohol-Gemische, wobei als Alkohole die niedermolekularen Alkohole Methanol, Ethanol, Isopropanol oder n-Propanol bevorzugt sind.

Die Reaktionstemperaturen können bei der Durchführung des erfindungsgemäßen Verfahrens (c) in einem größeren Bereich variiert werden. Im allgemeinen arbeitet man zwischen +20°C und 150°C, vorzugsweise zwischen +40°C und +120°C.

Zur Durchführung des erfindungsgemäßen Verfahrens (c) setzt man pro Mol 6-Alkylthio-3-(2,2-tetramethylenpropyl)-1,3,5-triazin-2,4-dion der Formel (Ic) im allgemeinen 1 bis 10 Mol, vorzugsweise 1 bis 7 Mol an Amin der Formel (IV) ein. Die Aufarbeitung erfolgt nach allgemein üblichen Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Verbindungen der Formel (I) neben einer deutlich besseren herbiziden Wirkung gegenüber wichtigen Schadpflanzen auch eine erheblich bessere Verträglichkeit gegenüber wichtigen Kulturpflanzen als die aus dem Stand der Technik bekannten Verbindungen und können zur selektiven Unkrautbekämpfung sowohl in dikotylen Kulturen wie z.B. Baumwolle, als auch in monokotylen Kulturen wie z.B. Hafer oder Mais eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen

Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage:

z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der

Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die erfindungsgemäßen Wirkstoffe der Formel (I) besitzen zum Teil auch eine fungizide Wirkung gegen Pyricularia oryzae an Reis sowie eine wurzelsystemische und nematizide Wirkung.

Die Herstellung und die Verwendung der erfindungsgemäßen Wirkstoffe geht aus den nachfolgenden Beispielen hervor.

## Herstellungsbeispiele

### Beispiel 1

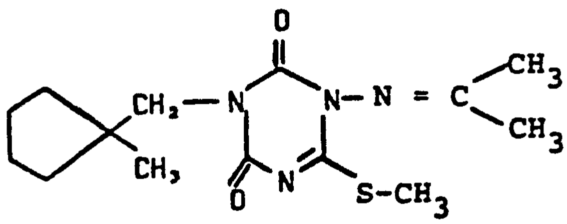

(1)

(Verfahren a)

Zu 53,0 g (0,15 Mol) N,N-Bisphenoxycarbonyl-(2,2-tetramethylen-propyl)-amin gibt man 23,9 g (0,15 Mol) Aceton-(S-methyl-isothiosemicarbazon) und erwärmt die Mischung auf 90°C. Nach Abklingen der schwach exothermen Reaktion rührt man noch 5 Stunden bei 100°C und destilliert dann die Hauptmenge des gebildeten Phenols im Vakuum ab. Der verbleibende Rückstand wird aus 1,1 Teilen Methanol umkristallisiert. Man erhält 33,9 g (72,9 % der Theorie) 1-Isopropylidenimino-3-(2,2-tetramethylen-propyl)-6-methylthio-1,2,3,4-tetrahydro-1,3,5-triazin-2,4-dion vom Schmelzpunkt 102-104°C.

### Herstellung der Ausgangsverbindungen

a) 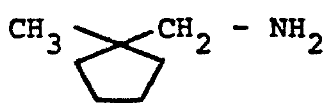

2395 g 1-Methyl-cyclopentyl-carbonitril werden nach Zusatz von 200 g Raney-Nickel und 2000 ml flüssigem Ammoniak bei 100 - 120°C und einem Druck von 150 bar hydriert. Man filtriert den Katalysator ab, dampft den

Ammoniak-Überschuß ab und fraktioniert den Rückstand an einer kurzen Füllkörperkolonne. Man erhält 2125 g (85,6 % der Theorie) 2,2-Tetramethylen-propylamin vom Siedepunkt 147-148°C bei 1010 mbar bzw. 46,5-47°C bei 18 mbar.

b) 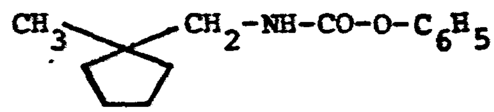

Zu einer Lösung von 347,4 g Chlorameisensäure-phenylester in 1110 ml Dichlormethan tropft man gleichzeitig bei 15-20°C 295 ml (250,8 g bzw. 2,22 Mol) 2,2-Tetramethylen-propylamin sowie 295 ml einer wäßrigen Natriumhydroxidlösung, die in dem angegebenen Volumen 88,8 g (2,22 Mol) Natriumhydroxid enthält. Nach beendeter Zugabe rührt man 2 Stunden bei Raumtemperatur, verdünnt mit 500 ml Wasser, trennt die organische Phase ab und dampft das Lösungsmittel ab. Man erhält als Rückstand 517 g (100 % der Theorie) N-(2,2-Tetramethylen-propyl)-carbamidsäure-phenylester vom Schmelzpunkt 58-60°C.

c) 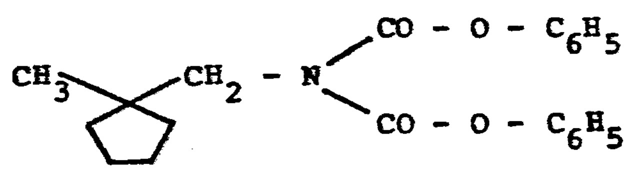

Eine Lösung von 517 g (2,22 Mol) N-(2,2-Tetramethylen-propyl)-carbamidsäure-phenylester in 540 ml Dichlormethan tropft man innerhalb 8 Stunden gleichmäßig unter die Oberfläche von 2780 ml (22,2 Mol) siedendem Chlorameisensäure-phenylester (Kp: 185-187°C), wobei gleichzeitig ein trockener Stickstoffstrom durch die Lösung

geleitet wird. Das Dichlormethan wird dabei über den Kopf eines auf 100°C temperierten Rückflußkühlers abdestilliert. Nach beendeter Zugabe rührt man eine weitere Stunde bei einer Sumpftemperatur von 187-190°C unter gleichzeitigem Durchleiten von Stickstoff, destilliert dann überschüssigen Chlorameisensäurephenylester im Wasserstrahlvakuum ab und destilliert den Rückstand an einer kurzen Füllkörperkolonne im Hochvakuum an, bis ein Siedepunkt von 164°C bei 0,008 mbar erreicht ist. Als Rückstand verbleiben 714 g Produkt mit einem GC-Gehalt von 97,1 % (Ausbeute 88,4 % der Theorie), welches beim Erkalten erstarrt.

Das reine N,N-Bisphenoxycarbonyl-(2,2-tetramethylen-propyl)-amin siedet bei 166°C / 0,008 mbar und schmilzt bei 65-66°C.

Beispiel 2

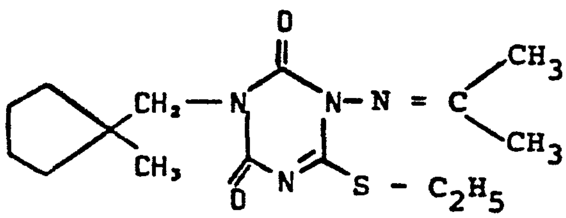

(2)

(Verfahren a)

Herstellung erfolgt analog zu Beispiel 1 unter Verwendung von Aceton-(S-ethyl-isothiosemicarbazon): Ausbeute 76 % der Theorie an 1-Isopropylidenimino-3-(2,2-tetramethylen-propyl)-6-ethylthio-1,2,3,4-tetrahydro-1,3,5-triazin-2,4-dion vom Schmelzpunkt 89-91°C.

Beispiel 3

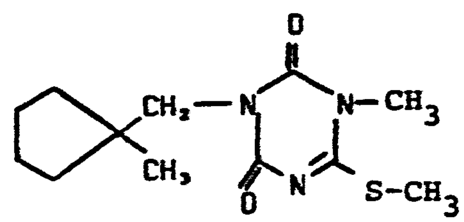

(3)

(Verfahren a)

In eine Suspension von 175,1 g (0,496 Mol) N,N-Bisphenoxycarbonyl-(2,2-tetramethylen-propyl)-amin und 115,1 g (0,496 Mol) N,S-Dimethyl-isothioharnstoff-hydroiodid in 1 Liter sek-Butanol gibt man 50,1 g (0,496 Mol) Triethylamin und kocht 4 Stunden unter Rückfluß. Man destilliert das Lösungsmittel im Vakuum ab, löst den Rückstand in 1 Liter Dichlormethan und schüttelt die Lösung zweimal mit je 500 ml Wasser, zweimal mit je 500 ml 1n Natronlauge und noch einmal mit Wasser aus. Der Eindampfrückstand der organischen Phase erstarrt beim Erkalten. Man erhält 130 g rohes 1-Methyl-3-(2,2-tetramethylen-propyl)-6-methylthio-1,2,3,4-tetrahydro-1,3,5-triazin-2,4-dion mit einem GC-Gehalt von 86,8 % (Ausbeute 89,4 % der Theorie). Die reine Substanz wird durch Umkristallisieren aus Cyclohexan erhalten: 90,7 g (68 % der Theorie) vom Schmelzpunkt 85-86°C.

Beispiel 4

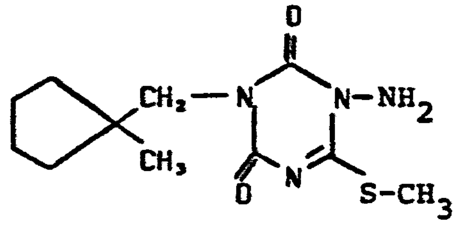

(4)

(Verfahren b)
Eine Lösung von 19,7 g (0,0635 Mol) 1-Isopropylidenimino-3-(2,2-tetramethylen-propyl)-6-methylthio-1,2,3,4-tetrahydro-1,3,5-triazin-2,4-dion in 70 ml Isopropanol versetzt man bei 60°C mit 0,6 g p-Toluolsulfonsäurehydrat und 2,3 ml Wasser. Sodann engt man bei einer Bad-Temperatur von 70°C und einem Druck von 250 mbar auf die Hälfte ein und kühlt anschließend auf 0°C, wobei das Reaktionsprodukt kristallisiert. Man erhält 13,1 g (76,4 % der Theorie) 1-Amino-3-(2,2-tetramethylen-propyl)-6-methylthio-1,2,3,4-tetrahydro-1,3,5-triazin-2,4-dion vom Schmelzpunkt 149-150°C.

Beispiel 5

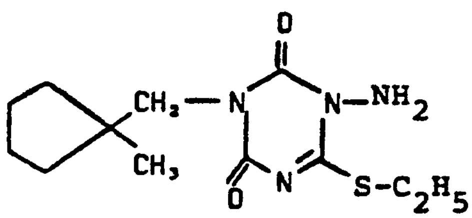

(5)

(Verfahren b)
Herstellung erfolgt analog zu Beispiel 4 unter Verwendung des Reaktionsproduktes von Beispiel 2.
Ausbeute 96 % der Theorie an 1-Amino-3-(2,2-tetramethylen-propyl)-6-ethylthio-1,2,3,4-tetrahydro-1,3,5-triazin-2,4-dion vom Schmelzpunkt 131-133°C.

Beispiel 6

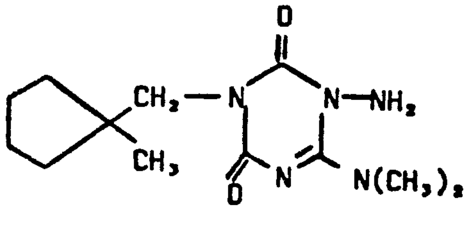

(6)

(Verfahren c)

Man rührt eine Suspension von 1206 g (4,25 Mol) 1-Amino-3-(2,2-tetramethylen-propyl)-6-ethylthio-1,2,3,4-tetrahydro-1,3,5-triazin-2,4-dion in 4600 g (48,6 Mol) 47,5 %iger wässriger Dimethylamin-Lösung 6 Stunden bei Raumtemperatur und zieht das überschüssige Dimethylamin sowie das gebildete Ethylmercaptan in eine auf -70°C gekühlte Vorlage bei einem Druck von 250-100 mbar ab, wobei sich der Reaktionskolben bis auf 0°C abkühlt. Man saugt bei -5°C ab, wäscht mehrfach mit Wasser, trocknet bei 70°C und erhält 1083 g (95,4 % der Theorie) 1-Amino-3-(2,2-tetramethylen-propyl)-6-dimethylamino-1,2,3,4-tetrahydro-1,3,5-triazin-2,4-dion vom Schmelzpunkt 134-136°C.

In entsprechender Weise erhält man die folgenden Verbindungen der Formel (I):

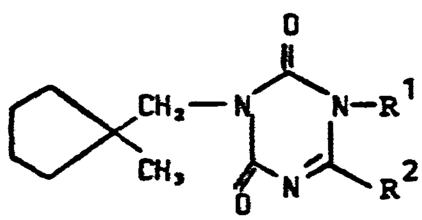

(I)

| Bsp.Nr. | $R^1$ | $R^2$ | Fp (°C) | Ausb.(% d. Th.) |
|---|---|---|---|---|
| (7) | $NH_2$ | $NH-CH_3$ | 177-178 | 94,0 |
| (8) | $CH_3$ | $NH-CH_3$ | 224-228 | 95,7 |
| (9) | $CH_3$ | $N(CH_3)_2$ | 144-145 | 87,2 |
| (10) | $CH_3$ | NH- 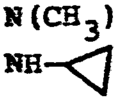 | 189-190 | 78,5 |

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der herbiziden Wirksamkeit gegenüber dem Stand der Technik zeigen in diesem Test z. B. die Verbindungen gemäß folgenden Herstellungsbeispielen: (6)

Als Vergleichssubstanz wurde die nachfolgend genannte Verbindung herangezogen: 1-Amino-6-ethylthio-3-neopentyl-1,2,3,4-tetrahydro-1,3,5-triazin-2,4-dion (commonname:Ametridione / bekannt aus DK-PS 136.067).

Patentansprüche

1) 3-(2,2-Tetramethylen-propyl)-1,3,5-triazin-2,4-dione der allgemeinen Formel (I)

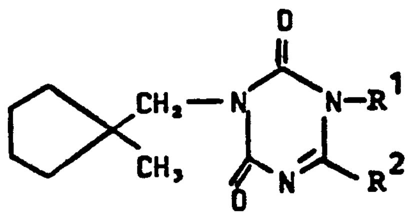

(I),

in welcher

$R^1$ für Alkyl, für Amino oder für einen Rest

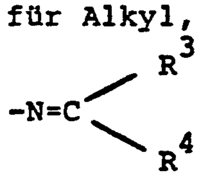

steht, wobei $R^3$ und $R^4$ unabhängig voneinander für Wasserstoff, für Alkyl oder für gegebenenfalls substituiertes Aryl stehen, oder gemeinsam für einen zweifach verknüpften Alkandiylrest stehen, und

$R^2$ für Alkylthio, Alkylamino, Alkenylamino, Cycloalkylamino oder Dialkylamino steht.

2. Verbindungen der allgemeinen Formel (I) gemäß Anspruch 1, dadurch gekennzeichnet, daß darin

$R^1$ für Amino, für geradkettiges oder verzweigtes Alkyl mit bis zu 4 Kohlenstoffatomen oder für einen Rest

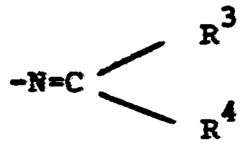 steht, wobei $R^3$ und $R^4$ jeweils unabhängig voneinander für Wasserstoff, für geradkettiges oder verzweigtes Alkyl mit bis zu 6 Kohlenstoffatomen oder für einfach oder mehrfach, gleich oder verschieden durch Halogen, Cyano, Nitro, sowie jeweils geradkettiges oder verzweigtes Alkyl, Alkoxy oder Alkylthio mit jeweils bis zu 4 Kohlenstoffatomen substituiertes Phenyl stehen oder gemeinsam für einen zweifach gebundenen Alkandiylrest mit 4 bis 6 Kohlenstoffatomen stehen und

$R^2$ für jeweils geradkettiges oder verzweigtes Alkylthio, Alkylamino oder Dialkylamino mit jeweils bis zu 6 Kohlenstoffatomen in den einzelnen Alkylteilen steht.

3. Verbindungen der allgemeinen Formel (I) gemäß Anspruch 1, dadurch gekennzeichnet, daß darin

$R^1$ für Methyl, Ethyl, Amino oder einen der Reste

$-N{=}C(CH_3)_2$, $-N{=}CH{-}C(CH_3)_3$, $-N{=}CH{-}C_6H_5$, -N= 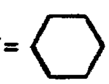

-N= 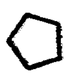 oder -N= 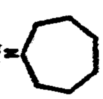 steht und

$R^2$ für Methylthio, Ethylthio, n- und i-Propylthio, n-, i-, s- und t-Butylthio, Methylamino, Ethylamino, Dimethylamino oder Diethylamino steht.

4) Verfahren zur Herstellung von 3-(2,2-Tetramethylen-propyl)-1,3,5-triazin-2,4-dionen der allgemeinen Formel (I) gemäß Anspruch 1, dadurch gekennzeichnet, daß man

(a) Isothioharnstoff-Derivate der Formel (II),

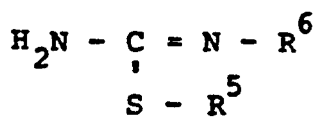

(II)

in welcher

$R^5$ für Alkyl steht und

$R^6$ für Alkyl oder für gegebenenfalls substituiertes Alkylidenimino bzw. Cycloalkylidenimino steht,

oder deren Hydrosalze mit (2,2-Tetramethylen-propyl)-amin-Derivaten der Formel (III)

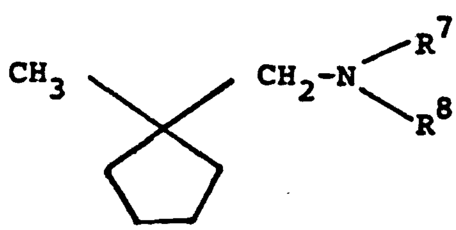

(III)

in welcher

$R^7$ und $R^8$ unabhängig voneinander jeweils (a) für gegebenenfalls substituiertes Aryloxycarbonyl oder (b) für Chlorcarbonyl stehen,

gegebenenfalls in Gegenwart eines Verdünnungsmittels und gegebenenfalls in Gegenwart eines Säurebindemittels umsetzt,

oder daß man

(b) die nach Verfahren (a) erhältlichen 1-Alkylidenimino-3-(2,2-tetramethylen-propyl)-1,3,5-triazin-2,4-dione der Formel (Ia)

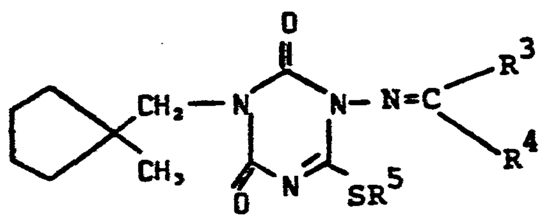

(Ia)

in welcher

$R^3$, $R^4$ und $R^5$ die oben angegebene Bedeutung haben,

in prinzipiell bekannter Weise mit wäßrigen Säuren, gegebenenfalls in Gegenwart eines Verdünnungsmittels und gegebenenfalls in Gegenwart eines Katalysators, umsetzt zu den 1-Amino-3-(2,2-tetramethylen-propyl)-1,3,5-triazin-2,4-dionen der Formel (Ib),

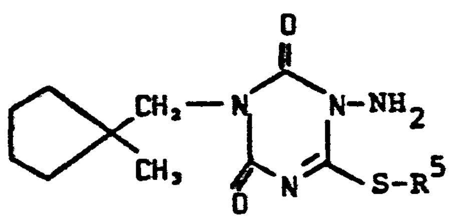

(Ib)

in welcher

$R^5$ die oben angegebene Bedeutung hat,

oder daß man

(c) die nach Verfahren (a) oder (b) erhältlichen 6-Alkylthio-3-(2,2-tetramethylen-propyl)-1,3,5-triazin-2,4-dione der Formel (Ic),

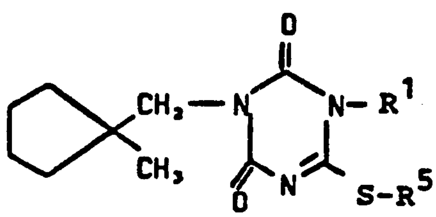

(Ic)

in welcher

$R^1$ und $R^5$ die oben angegebene Bedeutung haben,

mit Aminen der Formel (IV)

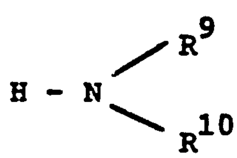

(IV)

in welcher

$R^9$ für Wasserstoff, Alkyl, Alkenyl oder Cycloalkyl steht und

$R^{10}$ für Alkyl steht,

0160208

gegebenenfalls in Gegenwart eines Verdünnungsmittels umsetzt zu den 6-Amino-3-(2,2-tetramethylen-propyl)-1,3,5-triazin-2,4-dionen der Formel (Id),

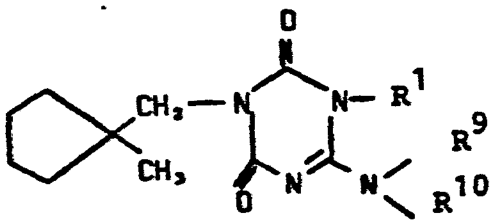

(Id)

in welcher

$R^1$, $R^9$ und $R^{10}$ die oben angegebene Bedeutung haben.

5) Herbizide Mittel, gekennzeichnet durch einen Gehalt an mindestens einem 3-(2,2-Tetramethylen-propyl)-1,3,5-triazin-2,4-dion der Formel (I) gemäß Anspruch 1.

6) Verwendung von 3-(2,2-Tetramethylen-propyl)-1,3,5-triazin-2,4-dionen der Formel (I) gemäß Anspruch 1 zur Bekämpfung von unerwünschtem Pflanzenwachstum.

7) Verfahren zur Herstellung von herbiziden Mitteln, dadurch gekennzeichnet, daß man 3-(2,2-Tetramethylen-propyl)-1,3,5-triazin-2,4-dione der Formel (I) gemäß Anspruch 1 mit Streckmitteln und/oder oberflächenaktiven Mitteln vermischt.

8) 1-Amino-3-(2,2-tetramethylen-propyl)-6-dimethylamino-1,2,3,4-tetrahydro-1,3,5-triazin-2,4-dion der Formel

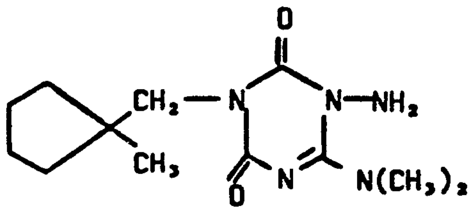

gemäß Anspruch 1.

Europäisches Patentamt

# EUROPÄISCHER RECHERCHENBERICHT

0160208
Nummer der Anmeldung

EP 85 10 3499

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | US-A-3 983 116 (K. LIN)<br>* Ansprüche 1,10,11; Spalte 26, Zeilen 65,66; Spalte 31, Zeile 46 - Spalte 32, Zeile 19 *<br>--- | 1-3,5-7 | C 07 D 251/38<br>C 07 D 251/46<br>A 01 N 43/64 |
| D,A | DE-A-2 254 200 (BAYER AG)<br>* Ansprüche; Seite 9, Zeile 13 - Seite 11, Zeile 24; Seite 19, Zeile 17 - Seite 24, Zeile 27 *<br>--- | 1-3,5-7 | |
| D,A<br>P | DE-A-3 241 114 (BAYER AG)<br>* Anspruch 1; Seiten 32-35, Tabellen 3,4 *<br>--- | 1-4 | |
| A | EP-A-0 058 895 (BAYER AG)<br>* Anspruch 1; Tabelle 1 *<br>--- | 1-4 | RECHERCHIERTE SACHGEBIETE (Int. Cl.4) |
| A | EP-A-0 034 751 (BAYER AG)<br>* Anspruch 1; Beispiel 1; Seiten 20-23 oben *<br>--- | 1-4 | A 01 N 43/64<br>C 07 D 251/00 |
| D,A | DE-A-3 147 735 (BAYER AG)<br>* Anspruch 1 *<br>--- | 4 | |
| D,A | DE-A-3 115 976 (BAYER AG)<br>* Seite 10, Zeile 13 - Seite 12; Tabelle 1, Beispiel 3, Verfahrensprokukt (I) *<br>----- | 4 | |

Der vorliegende Recherchenbericht wurde für alle Patentansprüche erstellt.

| Recherchenort | Abschlußdatum der Recherche | Prüfer |
|---|---|---|
| BERLIN | 03-07-1985 | HASS C V F |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82